(19) Europäisches Patentamt
European Patent Office
Office européen des brevets

(11) Veröffentlichungsnummer: **0 403 961 B1**

(12) **EUROPÄISCHE PATENTSCHRIFT**

(45) Veröffentlichungstag der Patentschrift: **02.03.94**

(51) Int. Cl.$^5$: **A61K 47/48**

(21) Anmeldenummer: **90111264.9**

(22) Anmeldetag: **14.06.90**

Die Akte enthält technische Angaben, die nach dem Eingang der Anmeldung eingereicht wurden und die nicht in dieser Patentschrift enthalten sind.

# (54) Magnetische Protein-Konjugate, Verfahren zu ihrer Herstellung und ihre Verwendung.

(30) Priorität: **19.06.89 DE 3919873**

(43) Veröffentlichungstag der Anmeldung:
**27.12.90 Patentblatt 90/52**

(45) Bekanntmachung des Hinweises auf die Patenterteilung:
**02.03.94 Patentblatt 94/09**

(84) Benannte Vertragsstaaten:
**AT BE CH DE DK ES FR GB GR IT LI LU NL SE**

(56) Entgegenhaltungen:
**EP-A- 0 125 995**

**JOURNAL OF IMMUNOLOGICAL METHODS, Band 24, Nr. 3/4, 1978, Seiten 321-336, Amsterdam, NL; E.S. RECTOR et al.: "A method for the preparation of protein-protein conjugates of predetermined composition"**

**THE LANCET, Band 1, Nr. 8368, 1984, Seiten 70-73, Londen, GB; J.G. TRELEAVEN et al.: "Removal of neuroblastoma cells from bone marrow with monoclonal antibodies conjugated to magnetic microspheres"**

(73) Patentinhaber: **BEHRINGWERKE Aktiengesellschaft
Postfach 1140
D-35001 Marburg(DE)**

(72) Erfinder: **Hermentin, Peter, Dr.
Salzköppel 9
D-3550 Marburg(DE)**
Erfinder: **Dönges, Reiner
Schöne Aussicht 1d
D-3554 Gladenbach(DE)**
Erfinder: **Franssen, Udo, Dr.
Salzköppel 11
D-3550 Marburg(DE)**
Erfinder: **Enssle, Karlheinz, Dr.
Salegrund 4
D-3550 Marburg(DE)**
Erfinder: **Friesen, Heinz-Jürgen, Dr.
Im Dorfe 4
D-3550 Marburg(DE)**

**EP 0 403 961 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**BIOCONJUGATE CHEMISTRY, Band 1, 1990, Seiten 411-418, Washington, US; P. HERMENTIN et al.: "Hinge-thiol coupling of monoclonal antibody to silanized iron oxide particles and evaluation of magnetic cell depletion"**

**Exp. Hematol. 17. 914-920 (1989), Biéva et al.**

**Bioconjugate Chemistry 1990, vol. 1, no. 6, p. 411-418, Hermentin et al.**

## Beschreibung

Die Erfindung betrifft magnetische Protein-Konjugate der Formel I,

$M-NH-CO-(CH_2)_n-S-P$ I

mit n = 1-6, bevorzugt mit n = 1 oder 2 und besonders bevorzugt mit n = 1, für welche gilt:
M ist ein dispergierbares, magnetisch reagierendes Material oder Partikel, welches Aminogruppen trägt, und
P ist ein Protein.

P kann ein Protein sein, in welchem eine oder mehrere Thiolgruppen entweder in natürlicher Weise vorhanden sind oder durch Reduktion von Disulfidbindungen erzeugt oder durch chemische Reaktion eingeführt wurden.

P ist insbesondere ein Immunglobulin oder Immunglobulin-Rest, bevorzugt ein monoklonaler Antikörper oder ein Fab-, Fab'- oder $F(ab')_2$- Fragment, ein Antigen oder ein Enzym-, Hormon-, Lectin- oder Wachstumsfaktorrest.

P ist bevorzugt ein monoklonaler Antikörper de IgG- oder IgM-Klasse, insbesondere ein monoklonaler Antikörper, welcher gegen ein Antigen gerichtet ist, welches in Körperflüssigkeiten oder wäßrigen Salzlösungen in gelöster Form vorhanden ist oder ein monoklonaler Antikörper, welcher gegen ein Antigen gerichtet ist, welches auf Zellen exprimiert ist, wobei die das Antigen exprimierenden Zellen insbesondere Zellen des myeloischen oder lymphatischen Systems, Zellen des peripheren Bluts, insbesondere B-Lymphozyten, T- Lymphozyten oder deren Vorläuferzellen oder Tumorzellen, insbesondere Tumorzellen des Knochenmarks sein können. Solche Zellen können auch Erythrozyten, Bakterien, Mykoplasmen oder Protozoen sein. Im Rahmen der Erfindung sollen aber auch Viren als Zellen verstanden werden.

M ist bevorzugt ein dispergierbares Partikel mit Metalloxid-Core und einem Aminogruppen tragenden Hüllmantel, wobei das Metalloxid-Core eine Gruppe paramagnetischer Substanzem einschließen kann, bevorzugt ein Partikel dessen Durchmesser zwischen etwa 0,1 $\mu$ und etwa 100 $\mu$, bevorzugt jedoch zwischen etwa 0,1 $\mu$ und etwa 1,5 $\mu$ liegt.

Die Erfindung betrifft ferner ein Verfahren zur Herstellung eines magnetischen Protein-Konjugats der allgemeinen Formel I sowie die Verwendung eines Konjugats der Formel I zur spezifischen Entfernung von Zellen oder löslichen Antigenen, Rezeptoren, Substraten, Co-Faktoren oder Kohlenhydrat-Determinanten aus wäßrigen Salzlösungen oder Körperflüssigkeiten sowie die Verwendung im Rahmen eines Diagnostikums bzw. als Diagnostikum sowie insbesondere die Verwendung zur Knochenmarks-Depletion oder zur HLA-Typisierung.

Eine Knochenmarkstransplantation ist unter anderem bei der Behandlung bestimmter Formen der Leukämie und der Panmyelopathie (Knochenmarksschwund) oftmals die einzige Möglichkeit der Therapie.

Bei Leukämien und gewissen lymphoiden Neoplasien werden die Patienten bisweilen einer Ganzkörperbestrahlung mit extrem hoher Dosis und/oder einer aggressiven Chemotherapie unterworfen. Bei einer derartigen Behandlung werden die normalen Stammzellen des Knochenmarks, die Vorläufer aller Blutzellen, vollständig zerstört. Dem Patienten wird daher Knochenmark eines geeigneten Spenders reinfundiert, von dem sich Zellen in den Knochenmarkräumen des Empfängers ansiedeln und so die Neuentwicklung des Blutbildungs- und Immunsystems ermöglichen. Dieses Verfahren nennt man allogene Knochenmarkstransplantation.

Verantwortlich für das hohe Risiko der allogenen Knochenmarkstransplantation sind unter anderem die dem Patienten im reinfundierten Knochenmark mitübertragenen T-Lymphozyten des Spenders, welche die Zellen des Empfängers als fremd erkennen und sie deshalb angreifen und zerstören. Diese für den Patienten oft lebensbedrohende Knochenmarksunverträglichkeit nennt man "graft-versus-host"-Reaktion oder "graft-versus-host"-Erkrankung (GVHD). Die Risiken dieser "graft-versus-host"-Erkrankung können zum einen dadurch vermindert werden, daß man dem Patienten nach Möglichkeit genau typisiertes Knochenmark besonders geeigneter Spender, meist aus dem Verwandtenkreis, reinfundiert. Zum anderen können sie aber auch dadurch reduziert werden, daß man unerwünschte Zellpopulationen, wie sie z. B. T-Lymphozyten des Spender-Knochenmarks darstellen können, vor der Reinfusion in den Patienten selektiv eliminiert. Diese Elimination von Spender-T-Zellen kann z. B. durch selektive Lysis der zu entfernenden Zellen in Gegenwart von Komplement oder durch selektive Abtötung der T-Zellen mit Hilfe sogenannter Immuntoxine oder durch ein anderes Verfahren, z. B. durch magnetische Zell-Depletion des Knochenmarks erfolgen.

Eine derartige Zell-Depletion von Knochenmark kann man auf relativ einfache Weise so durchführen, daß man das Knochenmark mit einem monoklonalen Antikörper von der Maus inkubiert, welcher z. B. spezifisch gegen die T-Zellen des Knochenmarks gerichtet ist und sich infolgedessen nur an die T-Zellen

bindet. Derartige, mit monoklonalen Antikörpern von der Maus belegte T-Zellen können nun in einem zweiten Schritt dadurch entfernt werden, daß man sie z. B. mit anti-Maus-Immunglobulin vom Kaninchen, welches an magnetische Partikel gebunden ist, inkubiert, wodurch die T-Lymphozyten in spezifischer Weise mit dem magnetischen Material belegt werden, so daß sie sich mit Hilfe eines Magneten aus dem Knochenmark entfernen lassen (s. hierzu Vartdal et al., Transplantation (1987), 43, 366-371 und die dort zitierte Literatur).

In analoger Weise lassen sich aus dem Knochenmark auch andere Zellpopulationen, etwa Tumorzellen, entfernen, was für die sogenannte autologe Knochenmarkstransplantation von Bedeutung ist (s. hierzu Kvalheim et al., Cancer Research (1987), 47, 846-851 und die dort zitierte Literatur). Dabei kann, wie von Kvalheim et al., ibid., beschrieben, der die Tumorzellen erkennende monoklonale Antikörper auch direkt an die magnetischen Partikel gebunden werden, so daß der oben erwähnte Zweitantikörper (Kaninchen anti-Maus) nicht mehr benötigt wird.

Das vorstehend beschriebene Verfahren zur Knochenmarks-Depletion mit Hilfe monoklonaler oder polyklonaler Antikörper, welche an magnetische Partikel gebunden sind, ist noch sehr jung und bedarf der weiteren Entwicklung und Erprobung. Hierfür geeignete magnetische Partikel stehen heute in verschiedenster Form käuflich zur Verfügung, und ihre Herstellung wurde mehrfach in der Patentliteratur beschrieben (s. z. B. Chagnon et al., EP 0125995 A2 (Priorität US 493991 vom 12.05.83), Advanced Magnetics, oder Ughelstad et al., WO 8303920 vom 10.11.83, SINTEF). Von diesen magnetischen Partikeln ist bekannt, daß sie aus einem Metalloxid-Core bestehen, in welchem paramagnetische Substanzen eingeschlossen sein können, und daß das Core von einem Hüllmantel umgeben ist, welcher reaktive Gruppen, wie z. B. Aminophenyl-, Amino-, Carboxyl-, Hydroxy- oder Sulfhydrylgruppen, tragen kann, welche zur Kopplung von Proteinen verwendet werden können (Chagnon et al., EP 0125995 A2).

Bekannt ist beispielsweise, daß Carboxylgruppen tragende Partikel in Gegenwart eines Kondensationsmittels mit Aminogruppen von Proteinen zur Reaktion gebracht werden können (Chagnon et al., EP 0125995 A2).

Bekannt ist ferner die Kopplung von Proteinen an Amino-gruppen tragende magnetische Partikel unter Verwendung von Glutaraldehyd, wobei die Kopplung jeweils über die Amino-gruppen erfolgt (Chagnon et al., EP 0125995 A2).

Bekannt ist weiterhin, daß sich Hydroxygruppen tragende Partikel durch Reaktion mit p-Toluolsulfonylchlorid aktivieren lassen, und daß solchermaßen aktivierte Partikel mit Aminogruppen von Proteinen zur Reaktion gebracht werden können (Kvalheim et al., Cancer Research (1987), 47, 846-851).

All diese Kopplungsverfahren haben gemeinsam, daß das Protein jeweils über seine freien Aminogruppen an die Partikel angeknüpft wird. Eine derartige Kopplung über Aminogruppen Kann jedoch bei monoklonalen Antikörpern von erheblichem Nachteil sein, weil hierbei bisweilen die Spezifität und Reaktivität der Antikörper beeinträchtigt wird. Dies ist eine Folge der Tatsache, daß sich die Aminogruppen bei einem Antikörper sozusagen statistisch über das ganze Molekül verteilen und daher auch in der Antigen-Bindungsstelle der Fab-Fragmente lokalisiert sind, was bei einer Kopplung über diese Aminogruppen einen Spezifitätsverlust bewirkt.

Es ist weiterhin bekannt, daß sich Antikörper auch ohne chemische Verknüpfung rein adsorptiv auf magnetische Partikel aufziehen lassen, wenn die Partikel aus einem Styrol-Divinylbenzol-Copolymerisat bestehen, welches Eisenoxid enthält, da sich Protein bekanntlich unspezifisch an Polystyrol anbindet.

Auch bei diesem Verfahren muß jedoch mit einer Beeinträchtigung der Antikörperspezifität und -reaktivität gerechnet werden. Ein weiterer gravierender Nachteil dieses Verfahrens besteht jedoch darin, daß sich adsorptiv gebundene Antikörper bei der Knochenmarks-Depletion wieder ablösen und so dem Patienten bei der Reinfusion des depletierten Knochenmarks mit verabreicht werden können, was insbesondere bei einem vorangegangenen Therapieversuch mit monoklonalen Antikörpern zu ernsten Nebenreaktionen führen könnte. Dieses Problem ist jedoch bekannt und soll durch kovalente Anknüpfung der Antikörper an die magnetischen Partikel überkommen werden.

Bekannt ist auch, daß magnetische Partikel auf Polystyrol-Basis den gravierenden Nachteil haben, daß sie zu Aggregation neigen und sich an Zellen auch unspezifisch anlagern.

Ausgehend von diesem Stand der Technik ist es die Aufgabe der vorliegenden Erfindung, eine Methode zu entwickeln, bei welcher monoklonale Antikörper an magnetische Partikel a) kovalent und b) nicht über ihre Aminogruppen gekoppelt werden. Aufgabe der vorliegenden Erfindung ist es daher, mit anderen Worten, ein Kopplungsverfahren zu finden, bei welchem die Antigen-Bindungsstelle des Antikörpers nicht verändert wird bzw. die Kopplung des Antikörpers abseits der Antigen-Bindungsstelle erfolgt.

Gelöst wird diese erfindungsgemäße Aufgabe durch Herstellung magnetischer Protein-Konjugate der Formel I

$M-NH-CO-(CH_2)_n-S-P$ I

Es wurde bereits vorgeschlagen, Aminogruppen tragende magnetische Partikel in magnetische Partikel zu überführen, welche als reaktive Gruppen Maleimido-Funktionen tragen und diese mit Proteinen zu konjugieren, welche Thiolgruppen besitzen, wobei die Thiolgruppen im Protein entweder bereits nativ vorhanden oder auf chemischem Wege eingeführt oder durch Reduktion vorhandener Disulfidbindungen erzeugt werden können.

Es wurde nun gefunden, daß sich magnetische Partikel, welche als reaktive Gruppen freie Aminogruppen tragen, auf einfache Weise auch in magnetische Partikel überführen lassen, welche als reaktive Gruppen Jodacetyl- oder Bromacetyl-Funktionen tragen. Derartige Partikel sind neu.

Weiterhin wurde gefunden, daß magnetische Partikel, welche Jodacetyl- oder Bromacetyl-Funktionen tragen, problemlos mit Proteinen konjugiert werden können, welche Thiolgruppen besitzen, wobei die Thiolgruppen im Protein entweder bereits nativ vorhanden oder auf chemischem Wege eingeführt oder durch Reduktion vorhandener Disulfidbindungen erzeugt werden können.

Insbesondere wurde gefunden, daß magnetische Partikel, welche Jodacetyl- oder Bromacetyl-Funktionen tragen, problemlos mit monoklonalen Antikörpern konjugiert werden können, wenn man die Interketten-Disulfid-Bindungen der Antikörper durch selektive Reduktion in freie SH-Gruppen überführt, welche mit den Jodacetyl- oder Bromacetyl-Funktionen der magnetischen Partikel unter Ausbildung einer Thioetherbindung zur Reaktion gebracht werden können. Diese Art der kopplung monoklonaler Antikörper an magnetische Partikel ist ebenfalls neu.

Überraschenderweise wurde gefunden, daß die Spezifität und Reaktivität der über Thioether-Bindungen an magnetische Partikel gekoppelte Antikörper voll erhalten bleibt, weil durch die Kopplung des Antikörpers über seine Hinge-Region seine Antigen-Bindungsstelle nicht verändert oder beeinträchtigt wird. Hierin liegt ein besonderer Vorteil der Erfindung gegenüber den bislang bekannten Kopplungsverfahren, bei denen die Antikörper, wie vorstehend beschrieben, entweder rein adsorptiv oder über Reaktion ihrer Aminogruppen auf magnetische Partikel aufgezogen werden, was sowohl die Spezifität als auch die Reaktivität der konjugierten Antikörper beeinträchtigen kann. Gegenüber der adsorptiven Kopplung besteht überdies bei der vorliegenden Erfindung der Vorteil, daß die Antikörper an die magnetischen Partikel chemisch gebunden sind und sich infolgedessen bei der Verwendung erfindungsgemäßer magnetischer Antikörper-Konjugate, z. B. zur Knochenmarks-Depletion, nicht von den Partikeln ablösen.

Weiterhin wurde gefunden, daß die bisweilen erfolgende unspezifische Adsorption von Antikörpern an die magnetischen Partikel durch Zusatz eines geeigneten Detergenz verhindert oder rückgängig gemacht werden kann.

Es wurde weiterhin gefunden, daß sich die erfindungsgemäßen magnetischen Antikörper-Konjugate, z. B. bei der Depletion von Knochenmark, wegen ihrer hohen Spezifität als besonders vorteilhaft erweisen.

Weiterhin wurde gefunden, daß sich die erfindungsgemäßen magnetischen Antikörper-Konjugate auch im Rahmen eines diagnostischen Verfahrens bzw. als Diagnostikum, insbesondere z. B. bei der HLA-Typisierung, wegen ihrer hohen Spezifität als vorteilhaft erweisen.

Die Herstellung erfindungsgemäßer magnetischer Antikörper-Konjugate ist nachfolgend für verschiedene monoklonale Antikörper, welche gegen Zellen des Knochenmarks gerichtet sind sowie für ein polyclonales Immunglobulin vom Kaninchen beispielhaft beschrieben; jedoch engen die genannten Beispiele die Erfindung nicht ein. Weiterhin ist die Verwendung der beispielhaft hergestellten magnetischen Antikörper-Konjugate zur Depletion von Zellen des Knochenmarks ebenfalls beispielhaft beschrieben, ohne die Verwendung auf die genannten Beispiele zu beschränken. Beispielhaft beschrieben ist auch die reduktive Spaltung der Disulfidbindung des Spacers durch Inkubation mit Dithiothreitol, ohne die Erfindung dadurch einzuschränken.

Verfahren zur Herstellung magnetischer Protein-Konjugate der Formel I:

Aminogruppen tragende magnetische Partikel M werden in einem geeigneten Lösungsmittel mit einer mit Aminogruppen reagierenden Halogenacyl-Spacer Verbindung der allgemeinen Formel II,

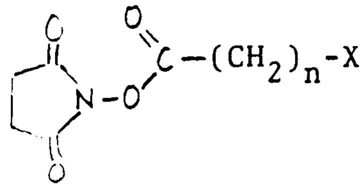

II

worin n = 1 oder 2 und X ein Chlor-, Brom- oder Jodatom ist, unter Ausbildung einer Amidbindung zu einer Verbindung der Formel III

$M-NH-CO-(CH_2)_n-X$ III

umgesetzt, welche schließlich in einem geeigneten wäßrigen salzhaltigen Lösungsmittel, welches Proteine nicht denaturiert, wie z. B. physiologische Kochsalzlösung oder eine mit Phosphat gepufferte Kochsalzlösung, mit einem Thiolgruppen tragenden Protein P, zu einer Verbindung der Formel I umgesetzt wird, worauf man die erhaltene Verbindung der Formel I von etwaig vorhandenem nicht Kovalent gebundenem Protein unter Zusatz eines geeigneten Detergenz, wie z. B. Tween, freiwäscht.

Geeignete Lösungsmittel zur Kopplung einer Verbindung der Formel II an magnetische Partikel müssen so beschaffen sein, daß die jeweils zur Kopplung verwendeten magnetischen Partikel in ihren physikalischen und magnetischen Eigenschaften, insbesondere in ihrer Größe, Dispergierbarkeit und Oberflächenbeschaffenheit, durch das verwendete Lösungsmittel nicht beeinträchtigt werden. Für magnetische Partikel, wie sie z. B. in EP 0125995 A2 oder WO 8303920 beschrieben sind, wurde als geeignetes Lösungsmittel z. B. eine Mischung aus Wasser und Dimethylformamid aufgefunden.

Bestimmung des Kopplungsgrades (µg Antikörper/mg Eisen):

Eisen wurde durch Atomabsorption und Stickstoff nach der Kjeldahl-Methode bestimmt. Die Werte für den gekoppelten Protein-Stickstoff wurden nach der Formel

$$\frac{\mu g\ P\text{-}N}{mg\ Fe} = \frac{\mu g\ Tot\text{-}N}{mg\ Fe}\ (Probe) - \frac{\mu g\ Tot\text{-}N}{mg\ Fe}\ (Kontrolle)$$

berechnet, wobei die Begriffe folgende Bedeutung haben:

P-N: Protein-Stickstoff
Tot-N.: Gesamt-Stickstoff
Fe: Eisen

Die an die Partikel gebundene Proteinmenge (µg Protein/mg Eisen) wurde aus der Proteinstickstoffmenge je mg Eisen durch Multiplikation mit dem Faktor 6.25 errechnet. Die errechneten Kopplungsraten sind in Tabelle 1 zusammengefaßt.

Verfahren zur Depletion von Zellen:

Eine Suspension eines zu depletierenden Zellgemischs in einer salzhaltigen, bevorzugt physiologischen wäßrigen Lösung oder in einer Körperflüssigkeit wird mit einer Verbindung der Formel I bei einer geeigneten Temperatur zwischen z. B. 0° C und 40° C, bevorzugt unter Schütteln, ebenfalls bevorzugt unter sterilen Bedingungen, über einen geeigneten Zeitraum inkubiert, und danach werden die magnetischen Partikel durch einen geeigneten Magneten aus der Lösung abgetrennt.

Geeignete Temperaturen sind beispielsweise 0° C, Raumtemperatur oder 37° C, bevorzugt jedoch Raumtemperatur. Die Zeitdauer der Inkubation richtet sich jeweils nach der verwendeten Inkubationstemperatur und nach der Bindungsreaktivität des Antikörpers und kann z. B. von wenigen Minuten bis zu z. B. 2 Stunden betragen. Bevorzugterweise wird z. B. bei Raumtemperatur z. B. über einen Zeitraum von 10 bis 20 Minuten inkubiert.

Verfahren zur Isolierung löslicher bioorganischer Moleküle:

Dieses Verfahren folgt im wesentlichen dem Verfahren zur Depletion von Zellen.

Beispiele:

Die nachfolgenden Beispiele dienen der näheren Erläuterung der Erfindung, schränken die Erfindung jedoch nicht ein.

Magnetische Partikel, welche in der beschriebenen Weise mit monoklonalen Antikörpern zur Reaktion gebracht wurden, werden nachfolgend als "Magnetobeads" bezeichnet, wobei ihre Spezifität jeweils durch Voranstellung der jeweiligen Antikörper-Bezeichnung wiedergegeben wird.

Beispiel 1:

Konjugation magnetischer Partikel gemäß EP 0125995 A2 mit N-Hydroxysuccinimidyl-jodacetat

4 x 300 µl einer käuflichen Suspension der magnetischen Partikel (BioMag$^R$, Advanced Magnetics) wurden jeweils 3 x mit je 10 ml Phosphat-gepufferter Kochsalzlösung pH 7.2 (PBS) gewaschen und jeweils in 3 ml PBS resuspendiert. Diese Suspensionen wurden jeweils mit einer frisch hergestellten Lösung von 10 mg N-Hydroxysuccinimidyl-jodacetat (NHIA; Rector et al., J. Immunol. Meth. (1978), 24, 321-336) in jeweils 2 ml trockenem Dimethylformamid versetzt und 1 h bei Raumtemperatur geschüttelt. Dann wurden die Partikel bei 3000 x g abzentrifugiert, jeweils 3 x mit je 10 ml PBS gewaschen und in a) 5 ml, b) 4,3 ml, c) 3,6 ml und d) 2,9 ml PBS, pH 7.2 resuspendiert.

Beispiel 2:

Konjugation magnetischer Partikel gemäß EP 0125995 A2 mit N-Hydroxysuccinimidyl-bromacetat

Die Konjugation erfolgte analog Beispiel 1 mit N-Hydroxy-succinimidyl-bromacetat (NHBrA), wobei die Herstellung des NHBrA in Analogie zur Herstellung von NHJA nach Rektor et al., ibid., erfolgte.

Beispiel 3:

Kopplung von polyklonalem Kaninchen anti-Maus-Immunglobulin (KAM) an gemäß Beispiel 1 aktivierte Partikel

3 mg polyklonales Kaninchen anti-Maus-Immunglobulin in Phosphat-gepufferter Kochsalzlösung (500 µl) wurde mit 3 mg Dithiothreitol versetzt und 30 min bei Raumtemperatur inkubiert. Der reduzierte Antikörper wurde mittels Gelfiltration über Sephadex G25 in Phosphat-gepufferter Kochsalzlösung pH 7.2 in einem Elutionsvolumen von 4,2 ml isoliert und zu den gemäß Beispiel 1 hergestellten Partikelsuspensionen a-d wie folgt zugesetzt:

a: kein Zusatz (Kontrolle)
b: Zusatz von 0,7 ml (ca. 0,5 mg Protein)
c: Zusatz von 1,4 ml (ca. 1,0 mg Protein)
d: Zusatz von 2,1 ml (ca. 1,5 mg Protein)

Die Mischungen (jeweils 5 ml) wurden jeweils 1 h unter Schütteln bei Raumtemperatur inkubiert. Dann wurden die Partikel bei 3000 x g abzentrifugiert, 3 x mit je 10 ml PBS gewaschen, in 5 ml PBS pH 7.2 resuspendiert und bei 4° C aufbewahrt. Die analytischen Daten sind in Tabelle 1 zusammengefaßt.

Beispiel 4:

Kopplung von polyklonalem Kaninchen anti-Maus-Immunglobulin (KAM) an gemäß Beispiel 2 aktivierte Partikel

Die Kopplung erfolgte analog Beispiel 3. Die analytischen Daten sind in Tabelle 1 zusammengefaßt.

Beispiel 5:

Kopplung des monoklonalen Antikörpers BMA 081 (anti-CD8; IgG2a) an gemäß Beispiel 1 aktivierte Partikel

2 mg BMA 081 in PBS (500 µl) wurden mit 1 mg Dithiothreitol versetzt und 30 min bei Raumtemperatur inkubiert. Der reduzierte Antikörper wurde mittels Gelfiltration über Sephadex G25 in Phosphat-gepufferter Kochsalzlösung pH 7.2 in einem Elutionsvolumen von 3,6 ml isoliert und zu den gemäß Beispiel 1 hergestellten Partikelsuspensionen a-d wie folgt zugesetzt:

a: kein Zusatz (Kontrolle)
b: Zusatz von 0,4 ml (ca. 0,2 mg Protein)
c: Zusatz von 1,2 ml (ca. 0,6 mg Protein)
d: Zusatz von 2,0 ml (ca. 1,0 mg Protein)

Die Mischungen (jeweils ca. 5 ml) wurden jeweils 1 h unter Schütteln bei Raumtemperatur inkubiert. Dann wurden die Partikel bei 3000 x g abzentrifugiert, 3 x mit je 10 ml PBS gewaschen, in 5 ml PBS pH 7.2 resuspendiert und bei 4° C aufbewahrt. Die analytischen Daten sind in Tabelle 1 zusammengefaßt.

Beispiel 6:

Kopplung des monoklonalen Antikörpers BMA 0110 (anti-CD2; IgG2b) an gemäß Beispiel 1 aktivierte Partikel

Die Kopplung erfolgte analog Beispiel 5. Die analytischen Daten sind in Tabelle 1 zusammengefaßt.

Beispiel 7:

Depletion von $CD8^+$-Zellen aus mononukleären Zellen mit gemäß Beispiel 5 hergestellten Magnetobeads

Mononukleäre Zellen (MNZ) wurden in an sich bekannter Weise (Boyum, Scand. J. Immunol. (1976), Suppl. 5, 9 - 15) über einen Ficoll-Gradienten aus frisch gespendetem Humanblut isoliert.

Für die Depletion wurden 3 x $10^7$ MNZ in 2 ml PBS mit 1 % BSA ((w/v), Seromed) in Plastik-Röhrchen (Falcon, Nr. 2051) mit 1 ml einer Suspension von 2 mg/ml Magnetobeads in PBS gemischt und 15 min bei Raumtemperatur unter dauerndem Schütteln inkubiert. Dann wurden die gemäß Beispiel 5 hergestellten Magnetobeads und die daran gebundenen Zellen mit Hilfe eines Permanentmagneten abgetrennt. Die in Suspension verbliebenen Zellen wurden bei 400 g pelletiert und in einem geeigneten Medium, z. B. PBS oder RPMI 1640, resuspendiert. Die Depletionseffizienz wurde mittels indirekter Immunfluoreszenz im Cytofluorograf (Ortho) bestimmt.

Hierzu wurden 1 x $10^6$ Zellen vor und nach Depletion einer bestimmten Zellpopulation mit 1 µg/ml Erstantikörper BMA 031 (Behringwerke AG) und danach mit 20 µg/ml Zweitantikörper (Kaninchen-anti-Maus-Immunglobulin, $F(ab)_2$-Fragment, FITC-markiert, Behringwerke) in an sich bekannter Weise markiert und im Cytofluorograph ausgewertet, wobei eine Depletionseffizienzen von über 95 % bestimmt wurden.

Tabelle 1

| Kopplungsgrade verschiedener hergestellter Magnetobeads | | | | |
|---|---|---|---|---|
| Beispiel | Antikörper | Isotyp | Kopplungsmethode | Kopplungsrate Protein/Eisen (µg/mg) |
| 3b | KAM | poly | NHJA | 74 |
| 3c | KAM | poly | NHJA | 136 |
| 3d | KAM | poly | NHJA | 171 |
| 4b | KAM | poly | NHBrA | 98 |
| 4c | KAM | poly | NHBrA | 143 |
| 4d | KAM | poly | NHBrA | 167 |
| 5b | BMA 081 | IgG2a | NHJA | 56 |
| 5c | BMA 081 | IgG2a | NHJA | 107 |
| 5d | BMA 081 | IgG2a | NHJA | 113 |
| 6b | BMA 0110 | IgG2b | NHJA | 76 |
| 6c | BMA 0110 | IgG2b | NHJA | 156 |
| 6d | BMA 0110 | IgG2b | NHJA | 183 |
| KAM: Kaninchen anti-Maus-Immunglobulin<br>poly: polyclonal<br>NHJA: N-Hydroxysuccinimidyl-jodacetat<br>NHBrA: N-Hydroxysuccinimidyl-bromacetat | | | | |

**Patentansprüche**

**Patentansprüche für folgende Vertragsstaaten : AT, BE, CH, DE, DK, FR, GB, IT, LI, LU, NL, SE**

**1.** Magnetisches Protein-Konjugat der allgemeinen Formel I,

$M\text{-}NH\text{-}CO\text{-}(CH_2)_n\text{-}S\text{-}P$ I

mit n = 1-6, bevorzugt mit n = 1 oder 2 und besonders bevorzugt mit n = 1, worin M ein dispergierbares, magnetisch reagierendes Material oder Partikel ist, welches Aminogruppen trägt, und P ein Protein ist.

**2.** Magnetisches Protein-Konjugat nach Anspruch 1,
**dadurch gekennzeichnet,**
daß die Thiolgruppe oder Thiolgruppen des Proteins P entweder in natürlicher Weise vorhanden sind oder durch Reduktion von Disulfidbindungen erzeugt oder durch chemische Reaktion in das Protein eingeführt werden.

**3.** Magnetisches Protein-Konjugat nach Anspruch 1,
**dadurch gekennzeichnet,**
daß P ein polyklonales Immunglobulin ist.

**4.** Magnetisches Protein-Konjugat nach Anspruch 1,
**dadurch gekennzeichnet,**
daß P ein monoklonaler Antikörper oder ein Fab-, Fab'- oder $F(ab')_2$-Fragment ist.

**5.** Magnetisches Protein-Konjugat nach Anspruch 1,
**dadurch gekennzeichnet,**
daß P ein Antigen oder ein Enzym-, Hormon-, Lectin- oder Wachstumsfaktorrest ist.

**6.** Magnetisches Protein-Konjugat nach Anspruch 4,
**dadurch gekennzeichnet**,
daß P einen monoklonalen Antikörper der IgG- oder IgM-Klasse darstellt.

7. Magnetisches Protein-Konjugat nach Anspruch 4,
**dadurch gekennzeichnet**,
daß P einen monoklonalen Antikörper darstellt, welcher gegen ein Antigen gerichtet ist, welches in wäßrigen Salzlösungen oder Körperflüssigkeiten in gelöster Form vorhanden ist.

8. Magnetisches Protein-Konjugat nach Anspruch 4,
**dadurch gekennzeichnet**,
daß P einen monoklonalen Antikörper darstellt, welcher gegen ein Antigen gerichtet ist, welches auf Zellen, insbesondere auf Zellen des myeloischen oder lymphatischen Systems oder des peripheren Bluts, insbesondere auf B-Lymphozyten, T-Lymphozyten oder deren Vorläuferzellen oder auf Tumorzellen, insbesondere auf Tumorzellen des Knochenmarks, exprimiert ist.

9. Magnetisches Protein-Konjugat nach Anspruch 4,
**dadurch gekennzeichnet**,
daß P einen monoklonalen Antikörper darstellt, welcher gegen ein Antigen gerichtet ist, welches auf Bakterien, Mykoplasmen oder Protozoen oder aber auf Viren exprimiert ist.

10. Magnetisches Protein-Konjugat nach Anspruch 1,
**dadurch gekennzeichnet**,
daß P ein Antigen darstellt.

11. Magnetisches Protein-Konjugat nach Anspruch 1,
**dadurch gekennzeichnet**,
daß M ein dispergierbares Partikel mit Metalloxid-Core und einem Aminogruppen tragenden Hüllmantel ist, wobei das Metalloxid-Core eine Gruppe paramagnetischer Substanzen einschließen kann.

12. Magnetisches Protein-Konjugat nach Anspruch 11,
**dadurch gekennzeichnet**,
daß der Durchmesser der Partikel zwischen etwa 0,1 µm und etwa 100 µm, bevorzugt jedoch zwischen etwa 0,1 µm und etwa 1,5 µm liegt.

13. Verfahren zur Herstellung eines magnetischen Protein-Konjugats der Formel I,

$M\text{-}NH\text{-}CO\text{-}(CH_2)_n\text{-}S\text{-}P$ I

**dadurch gekennzeichnet**,
daß man Aminogruppen tragende magnetische Partikel M mit einem mit Aminogruppen reagierenden Halogenoacyl Spacer der allgemeinen Formel II,

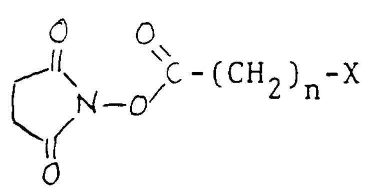

II

worin n = 1-6, bevorzugt n = 1 oder 2 und besonders bevorzugt n = 1 ist und X ein Chlor-, Brom- oder Jodatom ist, unter Ausbildung einer Amidbindung zu einer Verbindung der allgemeinen Formel III

$M\text{-}NH\text{-}CO\text{-}(CH_2)_n\text{-}X$ III

umsetzt, welche schließlich mit einem Thiolgruppen tragenden Protein P zu einer Verbindung der allgemeinen Formel I umgesetzt wird, worauf man die erhaltene Verbindung der Formel I von nicht kovalent gebundenem Protein freiwäscht.

14. Verbindung der Formel III,

M-NH-CO-$(CH_2)_n$-X III

worin n = 1-6, bevorzugt n = 1 oder 2 und besonders bevorzugt n = 1 ist und X ein Chlor-, Brom- oder Jodatom ist, worin M und n die in Anspruch 1 genannte Bedeutung haben und X ein Chlor-, Brom- oder Jodatom ist.

**15.** Verfahren zur Entfernung eines gelösten Antigens, Antikörpers, Rezeptors, Substrats, Co-Faktors oder einer Kohlenhydrat-Determinante aus wäßrigen Salzlösungen oder Körperflüssigkeiten,
**dadurch gekennzeichnet,**
daß man die Lösung mit einem geeigneten magnetischen Protein-Konjugat der Formel I

M-NH-CO-$(CH_2)_n$-S-P

mit n = 1-6, bevorzugt mit n = 1 oder 2 und besonders bevorzugt mit n = 1, worin M ein dispergierbares, magnetisch reagierendes Material oder Partikel ist, welches Aminogruppen trägt, und P ein Protein ist inkubiert und das magnetische Protein-Konjugat nach spezifischer Adsorption der zu entfernenden Komponente auf magnetischen Wege abtrennt und die spezifisch adsorbierte Komponente gegebenenfalls wieder vom magnetischen Protein-Konjugat abeluiert.

**16.** Verfahren zur Entfernung von Zellen aus wäßrigen Salzlösungen oder Körperflüssigkeiten,
**dadurch gekennzeichnet,**
daß man die Zellsuspension mit einem geeigneten magnetischen Protein-Konjugat der Formel I

M-NH-CO-$(CH_2)_n$-S-P

mit n = 1-6, bevorzugt mit n = 1 oder 2 und besonders bevorzugt mit n = 1, worin M ein dispergierbares, magnetisch reagierendes Material oder Partikel ist, welches Aminogruppen trägt, und P ein Protein ist inkubiert und das magnetische Protein-Konjugat nach spezifischer Adsorption der zu entfernenden Zellen auf magnetischem Wege abtrennt und die spezifisch adsorbierten Zellen oder Partikel gegebenenfalls wieder vom magnetischen Protein-Konjugat ablöst.

**17.** Verwendung eines magnetischen Protein-Konjugats nach Anspruch 1 zur spezifischen Entfernung von Zellen oder löslichen Antigenen, Rezeptoren, Substraten, Co-Faktoren oder Kohlenhydrat-Determinanten aus wäßrigen Salzlösungen oder Körperflüssigkeiten oder Verwendung im Rahmen eines diagnostischen Verfahrens bzw. als Diagnostikum.

**18.** Verwendung eines magnetischen Protein-Konjugats nach Anspruch 1 zur Entfernung von Zellen gemäß Anspruch 8 oder 9, bevorzugt zur Knochenmarks-Depletion, oder zur HLA-Typisierung.

**Patentansprüche für folgende Vertragsstaaten: ES, GR**

**1.** Verfahren zur Herstellung eines magnetischen Protein-Konjugats der Formel I,

M-NH-CO-$(CH_2)_n$-S-P I

dadurch gekennzeichnet, daß man Aminogruppen tragende magnetische Partikel M mit einem mit Aminogruppen reagierenden Maleimido- Spacer der allgemeinen Formel II,

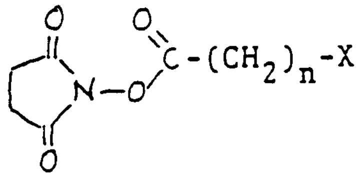 II

worin n = 1-6, bevorzugt n = 1 oder 2 und besonders bevorzugt n = 1 ist und X ein Chlor-, Brom- oder Jodatom ist, unter Ausbildung einer Amidbindung zu einer Verbindung der allgemeinen Formel III

M-NH-CO-$(CH_2)_n$-X III

umsetzt, welche schließlich mit einem Thiolgruppen tragenden Protein P zu einer Verbindung der allgemeinen Formel I umgesetzt wird, worauf man die erhaltene Verbindung der Formel I von nicht kovalent gebundenem Protein freiwäscht.

2. Verfahren nach Anspruch 1, dadurch gekennzeichnet, daß das Protein eine Thiolgruppe trägt.

3. Verfahren nach Anspruch 1, dadurch gekennzeichnet, daß P ein polyklonales Immunglobulin ist.

4. Verfahren nach Anspruch 1, dadurch gekennzeichnet, daß P ein monoklonaler Antikörper, ein Fab-, Fab'-oder $F(ab')_2$-Fragment, ein Antigen oder Enzym-, Hormon-, Lectin- oder Wachstumsfaktorrest ist.

5. Verfahren nach Anspruch 1, dadurch gekenzeichnet, daß P einen monoklonalen Antikörper der IgG- oder IgM-Klasse darstellt.

6. Verfahren nach Anspruch 1, dadurch gekenzeichnet, daß M ein dispergierbares Partikel mit Metalloxid-Core und einem Aminogruppen tragenden Hüllmantel ist.

7. Verfahren nach Anspruch 1, dadurch gekennzeichnet, daß M ein dispergierbares Partikel mit Metalloxid-Core und einem Aminogruppen tragenden Hüllmantel ist und der Durchmesser der Partikel zwischen etwa 0,1 $\mu$m und etwa 100 $\mu$m liegt.

## Claims

### Claims for the following Contracting States : AT, BE, CH, DE, DK, FR, GB, IT, LI, LU, NL, SE

1. A magnetic protein conjugate of the formula I

M-NH-CO-$(CH_2)_n$-S-P (I)

with n = 1-6, preferably with n = 1 or 2 and particularly preferably with n = 1, in which M is a dispersible, magnetically reacting material or particle which carries amino groups, and P is a protein.

2. A magnetic protein conjugate as claimed in claim 1, wherein the thiol group or thiol groups of the protein P are either present in the natural way or generated by reduction of disulfide linkages or introduced by chemical reaction into the protein.

3. A magnetic protein conjugate as claimed in claim 1, wherein P is a polyclonal immunoglobulin.

4. A magnetic protein conjugate as claimed in claim 1, wherein P is monoclonal antibody or an Fab, Fab' or $F(ab')_2$ fragment.

5. A magnetic protein conjugate as claimed in claim 1, wherein P is an antigen or a residue of an enzyme, hormone, lectin or growth factor.

6. A magnetic protein conjugate as claimed in claim 4, wherein P is a monoclonal antibody of the IgG or IgM class.

7. A magnetic protein conjugate as claimed in claim 4, wherein P is a monoclonal antibody which is directed against an antigen which is present in dissolved form in aqueous salt solutions or body fluids.

8. A magnetic protein conjugate as claimed in claim 4, wherein P is a monoclonal antibody which is directed against an antigen which is expressed on cells, especially on cells of the myeloid or lymphatic system or of the peripheral blood, especially on B lymphocytes, T lymphocytes or the precursor cells thereof or on tumor cells, especially on tumor cells of the bone marrow.

9. A magnetic protein conjugate as claimed in claim 4, wherein P is a monoclonal antibody which is directed against an antigen which is expressed on bacteria, mycoplasmas or protozoa or else on viruses.

10. A magnetic protein conjugate as claimed in claim 1, wherein P is an antigen.

11. A magnetic protein conjugate as claimed in claim 1, wherein M is a dispersible particle with a metal oxide core and an enveloping coat carrying amino groups, it being possible for a group of paramagnetic substances to be embedded in the metal oxide core.

12. A magnetic protein conjugate as claimed in claim 11, wherein the diameter of the particles is between about 0.1 µm and about 100 µm, but preferably between about 0.1 µm and about 1.5 µm.

13. A process for the preparation of a magnetic protein conjugate of the formula I,

M-NH-CO-$(CH_2)_n$-S-P I

which comprises magnetic particles M which carry amino groups being reacted with a haloacyl spacer which reacts with amino groups and has the formula II

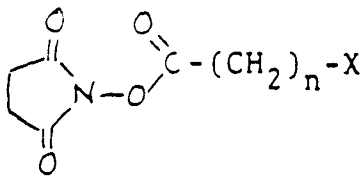

II

in which n = 1-6, preferably n = 1 or 2 and particularly preferably n = 1, and X is a chlorine, bromine or iodine atom, with the formation of an amide linkage to give a compound of the formula III

M-NH-CO-$(CH_2)_n$-X III

which is finally reacted with a protein P carrying thiol groups to give a compound of the formula I, after which the resulting compound of the formula I is washed free of non-covalently bonded protein.

14. A compound of the formula III

M-NH-CO-$(CH_2)_n$-X III

in which n = 1-6, preferably n = 1 or 2 and particularly preferably n = 1, and X is a chlorine, bromine or iodine atom, in which M has the meaning specified in claim 1.

15. A method for removing a dissolved antigen, antibody, receptor, substrate, cofactor or carbohydrate determinant from aqueous salt solutions or body fluids, which comprises the solution being incubated with a suitable magnetic protein conjugate of the formula I

M-NH-CO-$(CH_2)_n$-S-P I

with n = 1-6, preferably with n = 1 or 2 and particularly preferably with n = 1, in which M is a dispersible, magnetically reacting material or particle which carries amino groups, and P is a protein, and, after specific adsorption of the component which is to be removed, the magnetically protein conjugate being separated out by magnetic means, and the specifically adsorbed component being, where appropriate, eluted again from the magnetic protein conjugate.

16. A method for removing cells from aqueous salt solutions or body fluids, which comprise the cell suspension being incubated with a suitable magnetic protein conjugate of the formula I

M-NH-CO-$(CH_2)_n$-S-P (I)

with n = 1-6, preferably with n = 1 or 2 and particularly preferably with n = 1, in which M is a dispersible, magnetically reacting material or particle which carries amino groups, and P is a protein, and, after specific adsorption of the cells which are to be removed, the magnetic protein conjugate being separated out by magnetic means, and the specifically adsorbed cells or particles being, where appropriate, detached again from the magnetic protein conjugate.

**17.** The use of a magnetic protein conjugate as claimed in claim 1 for the specific removal of cells or soluble antigens, receptors, substrates, cofactors or carbohydrate determinants from aqueous salt solutions or body fluids or the use as part of a diagnostic method or as a diagnostic aid.

**18.** The use of a magnetic protein conjugate as claimed in claim 1 for removing cells as claimed in claim 8 or 9, preferably for bone marrow depletion, or for HLA typing.

**Claims for the following Contracting States : ES, GR**

**1.** A process for the preparation of a magnetic protein conjugate of the formula I,

$M\text{-}NH\text{-}CO\text{-}(CH_2)_n\text{-}S\text{-}P$ I

which comprises magnetic particles M which carry amino groups being reacted with a haloacyl spacer which reacts with amino groups and has the formula II

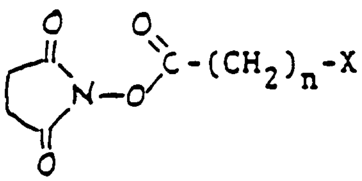

II

in which n = 1-6, preferably n = 1 or 2 and particularly preferably n = 1, and X is a chlorine, bromine or iodine atom, with the formation of an amide linkage to give a compound of the formula III

$M\text{-}NH\text{-}CO\text{-}(CH_2)_n\text{-}X$ III

which is finally reacted with a protein P carrying thiol groups to give a compound of the formula I, after which the resulting compound of the formula I is washed free of non-covalently bonded protein.

**2.** The process as claimed in claim 1, wherein the protein carries a thiol group.

**3.** The process as claimed in claim 1, wherein P is a polyclonal immunoglobulin.

**4.** The process as claimed in claim 1, wherein P is a monoclonal antibody, an Fab, Fab' or $F(ab')_2$ fragment, an antigen or a residue of an enzyme, hormone, lectin or growth factor.

**5.** The process as claimed in claim 1, wherein P is a monoclonal antibody of the IgG or IgM class.

**6.** The process as claimed in claim 1, wherein M is a dispersible particle with a metal oxide core and an enveloping coat carrying amino groups.

**7.** The process as claimed in claim 1, wherein M is a dispersible particle with a metal oxide core and an enveloping coat carrying amino groups and the diameter of the particles is between about 0.1 µm and about 100 µm.

**Revendications**

**Revendications pour les Etats contractants suivants : AT, BE, CH, DE, DK, FR, GB, IT, LI, LU, NL, SE**

**1.** Conjugué protéique magnétique de formule générale I,

$M\text{-}NH\text{-}CO\text{-}(CH_2)_n\text{-}S\text{-}P$ I

avec n = 1 à 6, de préférence, avec n = 1 ou 2, et mieux encore, n = 1, dans laquelle M est une particule ou un matériau dispersable, réagissant magnétiquement, qui porte des groupes amino, et P est une protéine.

**2.** Conjugué protéique magnétique selon la revendication 1, caractérisé en ce que le ou les groupe(s) thiol de la protéine P est (sont) présent(s) à l'état naturel ou est (sont) obtenu(s) par réduction de liaisons disulfure ou est (sont) introduit(s) dans la protéine par réaction chimique.

**3.** Conjugué protéique magnétique selon la revendication 1, caractérisé en ce que P est une immunoglobuline polyclonale.

**4.** Conjugué protéique magnétique selon la revendication 1, caractérisé en ce que P est un anticorps monoclonal ou un fragment Fab ou Fab' ou $F(ab')_2$.

**5.** Conjugué protéique magnétique selon la revendication 1, caractérisé en ce que P est un antigène ou un résidu d'enzyme, d'hormone, de lectine ou de facteur de croissance.

**6.** Conjugué protéique magnétique selon la revendication 4, caractérisé en ce que P représente un anticorps monoclonal de la classe des IgG ou des IgM.

**7.** Conjugué protéique magnétique selon la revendication 4, caractérisé en ce que P représente un anticorps monoclonal dirigé contre un antigène qui est présent, sous forme dissoute, dans des solutions salines aqueuses ou dans des liquides corporels.

**8.** Conjugué protéique magnétique selon la revendication 4, caractérisé en ce que P représente un anticorps monoclonal dirigé contre un antigène qui est exprimé dans des cellules, en particulier, dans des cellules du système myéloïque ou lymphatique ou du sang périphérique, en particulier, dans des lymphocytes B, des lymphocytes T ou des cellules-précurseurs de ceux-ci, ou dans des cellules tumorales, en particulier, dans des cellules tumorales de la moëlle osseuse.

**9.** Conjugué protéique magnétique selon la revendication 4, caractérisé en ce que P représente un anticorps monoclonal dirigé contre un antigène qui est exprimé dans des bactéries, des mycoplasmes ou des protozoaires, ou encore dans des virus.

**10.** Conjugué protéique magnétique selon la revendication 1, caractérisé en ce que P représente un antigène.

**11.** Conjugué protéique magnétique selon la revendication 1, caractérisé en ce que M est une particule dispersable dotée d'un noyau d'oxyde métallique et d'une enveloppe extérieure portant des groupes amino, le noyau d'oxyde métallique pouvant inclure un groupe de substances paramagnétiques.

**12.** Conjugué protéique magnétique selon la revendication 11, caractérisé en ce que le diamètre de la particule est compris entre environ 0,1 $\mu m$ et environ 100 $\mu m$, mais, de préférence, entre environ 0,1 $\mu m$ et 1,5 $\mu m$.

**13.** Procédé pour préparer un conjugué protéique magnétique de formule générale I,

$M\text{-}NH\text{-}CO\text{-}(CH_2)_n\text{-}S\text{-}P$ I

caractérisé en ce que l'on fait réagir une particule magnétique M, portant des groupes amino, avec un espaceur halogénoacyle, réagissant avec les groupes amino, de formule générale II,

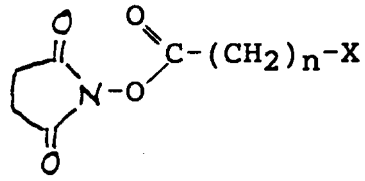

II

dans laquelle n = 1 à 6, de préférence, n = 1 ou 2, et mieux encore, n = 1, et X est un atome de chlore, d'iode ou de brome, en formant une liaison amide, pour obtenir un composé de formule générale III,

$M-NH-CO-(CH_2)_n-X$ III

que l'on fait ensuite réagir avec une protéine P portant des groupes thiol, pour obtenir un composé de formule générale I, après quoi on sépare le composé de formule I obtenu de la protéine qui n'est pas liée par covalence.

**14.** Composé de formule III,

$M-NH-CO-(CH_2)_n-X$ III

dans laquelle n = 1 à 6, de préférence, n = 1 ou 2, et mieux encore, n = 1, X est un atome de chlore, d'iode ou de brome, et M et n ont les significations données dans la revendication 1.

**15.** Procédé pour isoler un antigène, un anticorps, un récepteur, un substrat, un co-facteur ou un déterminant glucidique, à l'état dissous, de solutions salines aqueuses ou de liquides corporels, caractérisé en ce que l'on incube la solution avec un conjugué protéique magnétique approprié de formule I,

$M-NH-CO-(CH_2)_n-S-P$ I

avec n = 1 à 6, de préférence, avec n = 1 ou 2, et mieux encore, n = 1, dans laquelle M est une particule ou un matériau dispersable, réagissant magnétiquement, qui porte des groupes amino, et P est une protéine, et, après avoir adsorbé les composants à isoler de façon spécifique, on sépare le conjugué protéique magnétique, par voie magnétique et, éventuellement, on élue encore les composants adsorbés de façon spécifique pour les séparer du conjugué protéique magnétique.

**16.** Procédé pour isoler des cellules de solutions salines aqueuses ou de liquides corporels, caractérisé en ce que l'on incube la suspension cellulaire avec un conjugué protéique magnétique approprié de formule I,

$M-NH-CO-(CH_2)_n-S-P$ I

avec n = 1 à 6, de préférence, avec n = 1 ou 2, et mieux encore, n = 1, dans laquelle M est une particule ou un matériau dispersable, réagissant magnétiquement, qui porte des groupes amino, et P est une protéine, et, après avoir adsorbé les cellules à isoler de façon spécifique, on sépare le conjugué protéique magnétique, par voie type magnétique et, éventuellement, on détache encore les cellules ou particules adsorbées de façon spécifique pour les séparer du conjugué protéique magnétique.

**17.** Utilisation d'un conjugué protéique magnétique selon la revendication 1 pour l'isolement spécifique de cellules ou d'antigènes, de récepteurs, de substrats, de co-facteurs ou de déterminants glucidiques solubles, de solutions salines aqueuses ou de liquides corporels, ou utilisation dans le cadre d'un procédé de diagnostic et en tant qu'agent de diagnostic.

**18.** Utilisation d'un conjugué protéique magnétique selon la revendication 1 pour l'isolement de cellules selon la revendication 8 ou 9, de préférence, pour la déplétion de la moëlle osseuse ou pour la caractérisation de HLA.

**Revendications pour les Etats contractants suivants : ES, GR**

**1.** Procédé pour préparer un conjugué protéique magnétique de formule générale I,

$M\text{-}NH\text{-}CO\text{-}(CH_2)_n\text{-}S\text{-}P$ I

caractérisé en ce que l'on fait réagir une particule magnétique M, portant des groupes amino, avec un espaceur maléimido, réagissant avec les groupes amino, de formule générale II,

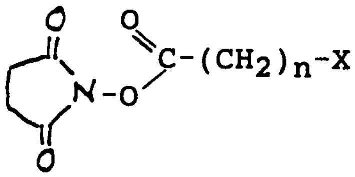

II

dans laquelle n = 1 à 6, de préférence, n = 1 ou 2, et mieux encore, n = 1, et X est un atome de chlore, d'iode ou de brome, en formant une liaison amide, pour obtenir un composé de formule générale III,

$M\text{-}NH\text{-}CO\text{-}(CH_2)_n\text{-}X$ III

que l'on fait ensuite réagir avec une protéine P portant des groupes thiol, pour obtenir un composé de formule générale I, après quoi on purifie le composé de formule I obtenu de la protéine qui n'est pas liée par covalence.

**2.** Procédé selon la revendication 1, caractérisé en ce que la protéine porte un groupe thiol.

**3.** Procédé selon la revendication 1, caractérisé en ce que P est une immunoglobuline polyclonale.

**4.** Procédé selon la revendication 1, caractérisé en ce que P est un anticorps monoclonal, un fragment Fab ou Fab' ou $F(ab')_2$, un antigène ou un résidu d'enzyme, d'hormone, de lectine ou de facteur de croissance.

**5.** Procédé selon la revendication 1, caractérisé en ce que P représente un anticorps monoclonal de la classe des IgG ou des IgM.

**6.** Procédé selon la revendication 1, caractérisé en ce que M est une particule dispersable dotée d'un noyau d'oxyde métallique et d'une enveloppe extérieure portant des groupes amino.

**7.** Procédé selon la revendication 1, caractérisé en ce que M est une particule dispersable dotée d'un noyau d'oxyde métallique et d'une enveloppe extérieure portant des groupes amino et le diamètre de la particule est compris entre environ 0,1 µm et environ 100 µm.